# EUROPEAN PATENT APPLICATION

(11) **EP 4 793 910 A1**
(43) Date of publication of application: **19.08.2026**
(21) Application number: 26151914.4
(22) Date of filing: 14.01.2026
(51) Int. Cl.: G06V 30/26, G06V 10/74, G06V 20/40, G06V 40/20, G16H 10/60, G16H 30/40, G06V 10/82, G06F 40/169, G06F 40/174, G06F 40/20, G06F 40/279, G10L 15/26, G16H 40/63

(54) **METHOD AND APPARATUS FOR ENTERING MEDICAL INFORMATION, ELECTRONIC DEVICE, AND READABLE STORAGE MEDIUM**

(30) Priority: 13.02.2025 CN 202510157747
(71) Applicant: Peking Union Medical College Hospital, Chinese Academy of Medical Sciences & Peking Union Medical College, Beijing 100730 (CN); Digital Health China Technologies Co., Ltd., Beijing 100080 (CN)
(72) Inventor: GUO, Jian, Beijing, 100730 (CN); ZHANG, Shuyang, Beijing, 100730 (CN); JIN, Ye, Beijing, 100730 (CN); ZHANG, Wen, Beijing, 100730 (CN); GONG, Mengchun, Beijing, 100080 (CN); SHI, Wenzhao, Beijing, 100080 (CN); ZHENG, Xihong, Beijing, 100080 (CN); XIA, Peng, Beijing, 100730 (CN); PENG, Linyi, Beijing, 100730 (CN); LIU, Peng, Beijing, 100730 (CN)
(74) Representative: Lapienis, Juozas

(57) **Abstract**

The present invention provides a method and an apparatus for entering medical information, an electronic device, and a readable storage medium, wherein the method includes: acquiring a medical document photo obtained by photographing text in a medical document by a camera located in a ward; calling, according to photographing time information and photographing location information about the medical document photo, recorded audio data and recorded video data used for medical document generation; extracting first document information from the medical document photo using OCR text recognition technology; correcting the first document information based on the recorded audio data and the recorded video data; and entering the corrected first document information into a database. The accuracy of information entry is improved by the method.

## Description

### TECHNICAL FIELD

The present invention relates to the field of medical technology, and in particular to a method and an apparatus for entering medical information, an electronic device, and a readable storage medium.

### BACKGROUND

During clinical medical practice, data entry is an important step that is both time-consuming and prone to errors. A relatively traditional approach involves filling CRF (case report form) forms with case data by means of manual entry. This approach not only requires a large amount of workload, but also is prone to errors during data entry, which affects data accuracy.

In order to improve the efficiency and accuracy of data entry, some technology teams have developed and started using OCR (optical character recognition) technology in data entry. They scan or photograph medical documents to acquire images of the documents, and then use OCR technology to recognize text in the images to acquire recognition results.

### SUMMARY

In view of this, an object of the present invention is to provide a method and apparatus for entering medical information, an electronic device, and a readable storage medium to improve the accuracy of information entry.

In a first aspect, an embodiment of the present invention provides a method for entering medical information, comprising:
acquiring a medical document photo obtained by photographing text in a medical document by a camera located in a ward;
calling, according to photographing time information and photographing location information about the medical document photo, recorded audio data and recorded video data used for medical document generation;
extracting first document information from the medical document photo using OCR text recognition technology;
correcting the first document information based on the recorded audio data and the recorded video data; and
entering the corrected first document information into a database.

In combination with the first aspect, an embodiment of the present invention provides a first possible implementation of the first aspect, wherein the camera is a portable camera set on a body of a doctor; and the acquiring a medical document photo obtained by photographing text in a medical document by a camera located in a ward comprises:
capturing a photo at a predetermined time interval to obtain a candidate photo;
performing foreground extraction on the candidate photo to extract a current foreground image from the candidate photo, a handheld medical document being included in the current foreground image;
calculating a foreground image similarity between the current foreground image and a pre-acquired historical foreground image; and
using the current foreground image as a medical document photo in response to the foreground image similarity being less than a preset similarity.

In combination with the first aspect, an embodiment of the present invention provides a second possible implementation of the first aspect, wherein the calling, according to photographing time information and photographing location information about the medical document photo, recorded audio data and recorded video data used for medical document generation comprises:
determining, according to the photographing location information about the medical document photo, a candidate room where the medical document photo is captured;
calculating a score of a reference picture of each candidate room according to the medical document photo by means of visual positioning;
determining a candidate room with a score exceeding a preset value as a target room; and
selecting, from video data recorded in the target room, a target recorded audio segment as recorded audio data to be called for the medical document generation, and a target video segment as recorded video data to be called for the medical document generation, according to the photographing time information about the medical document photo.

In combination with the first aspect, an embodiment of the present invention provides a third possible implementation of the first aspect, wherein the correcting the first document information based on the recorded audio data and the recorded video data comprises:
performing speech recognition on the recorded audio data to obtain speech-recognized text;
analyzing body movements of a person in the recorded video data to obtain body movement characteristic data;
extracting referential second document information corresponding to the speech-recognized text from a pre-established historical speech-recognized text - referential second document information comparison table based on the speech-recognized text;
extracting referential third document information corresponding to the body movement characteristic data from a pre-established historical body movement characteristic data - referential third document information comparison table based on the body movement characteristic data; and
correcting the first document information based on the referential second document information and the referential third document information.

In combination with the second possible implementation of the first aspect, an embodiment of the present invention provides a fourth possible implementation of the first aspect, wherein the entering the corrected first document information into a database comprises:
if there are multiple pieces of corrected first document information, retrieving medical information of a patient corresponding to the medical document photo;
displaying the medical information and the multiple pieces of corrected first document information in the terminal of the doctor to select one of the multiple pieces of corrected first document information as final first document information according to a selection instruction received by the terminal of the doctor; and
storing the final first document information in the database and updating the historical speech-recognized text - referential second document information comparison table and the historical body movement characteristic data - referential third document information comparison table based on the corrected final first document information.

In a second aspect, an embodiment of the present invention further provides an apparatus for entering medical information, including:
an acquisition module configured to acquire a medical document photo obtained by photographing text in a medical document by a camera located in a ward;
a calling module configured to call, according to photographing time information and photographing location information about the medical document photo, recorded audio data and recorded video data used for medical document generation;
an extraction module configured to extract first document information from the medical document photo using OCR text recognition technology;
a correction module configured to correct the first document information based on the recorded audio data and the recorded video data; and
an entry module configured to enter the corrected first document information into a database.

In a third aspect, an embodiment of the present invention further provides an electronic device, including: a processor, a memory, and a bus, wherein the memory stores machine-readable instructions executable by the processor, and when the electronic device is running, the processor communicates with the memory via the bus, and the machine-readable instructions are executed by the processor to execute the steps in any of the above possible implementations in the first aspect.

In a fourth aspect, an embodiment of the present invention further provides a computer-readable storage medium, wherein a computer program is stored on the computer-readable storage medium, and the computer program is run by a processor to execute the steps in any of the above possible implementations in the first aspect.

In the method for entering medical information according to an embodiment of the present invention, first document information in the form of text extracted from a medical document photo is corrected by using recorded audio data and recorded video data produced during capturing of the medical document photo, so that the accuracy of text information which is originally directly entered into the database is further enhanced, and the accuracy of the information is ensured.

In order to enable clearer and easier understanding of the above objects, features, and advantages of the present invention, preferred embodiments will be described in detail below by way of example with reference to the drawings.

### BRIEF DESCRIPTION OF THE DRAWINGS

In order to more clearly illustrate the technical solutions of embodiments of the present invention, drawings required to be used in the embodiments will be described briefly below. It is understood that the drawings below are merely illustrative of some embodiments of the present invention and thus should not be considered as limiting its scope. It will be appreciated by those of ordinary skill in the art that other relevant drawings can be obtained according to these drawings without any inventive effort.
FIG. 1 shows a flowchart of a method for entering medical information according to an embodiment of the present invention.
FIG. 2 shows a flowchart of another method for entering medical information according to an embodiment of the present invention.
FIG. 3 shows a schematic structural diagram of an apparatus for entering medical information according to an embodiment of the present invention.
FIG. 4 shows a schematic structural diagram of an electronic device according to an embodiment of the present invention.

### DETAILED DESCRIPTION OF THE EMBODIMENTS

In order to further clarify the objects, technical solutions, and advantages of the embodiments of the present invention, the technical solutions of the embodiments of the present invention will be described below clearly and completely with reference to the drawings of the embodiments of the present invention. It is apparent that the embodiments to be described are some, but not all of the embodiments of the present invention. Generally, the components of the embodiments of the present invention, as described and illustrated in the figures herein, may be arranged and designed in a wide variety of different configurations. Thus, the following detailed description of the embodiments of the present invention, as represented in the figures, is not intended to limit the scope of the present invention as claimed, but is merely representative of selected embodiments of the present invention. All the other embodiments obtained by those of ordinary skill in the art in light of the embodiments of the present invention without inventive efforts will fall within the scope of the present invention as claimed.

In the related art, when inquiring after an inpatient, a doctor usually readily records information on inquiry, such as the physical condition, recovery state, medication adjustment mode of the inpatient, and the like. After the information is obtained, the information written by the doctor may be entered into a system using OCR technology for subsequent viewing. However, when using OCR for text entry, misrecognition often occurs for two main reasons. Firstly, when a doctor inquires after a patient in a ward, the doctor usually uses a pad in his hand as a support and then writes on the pad, rather than recording in front of a desk, so that the standard of writing is much worse. Secondly, the doctor writes relatively fast and cursively, thus the written text is relatively arbitrary and can hardly be accurately recognized using a general OCR recognition technology.

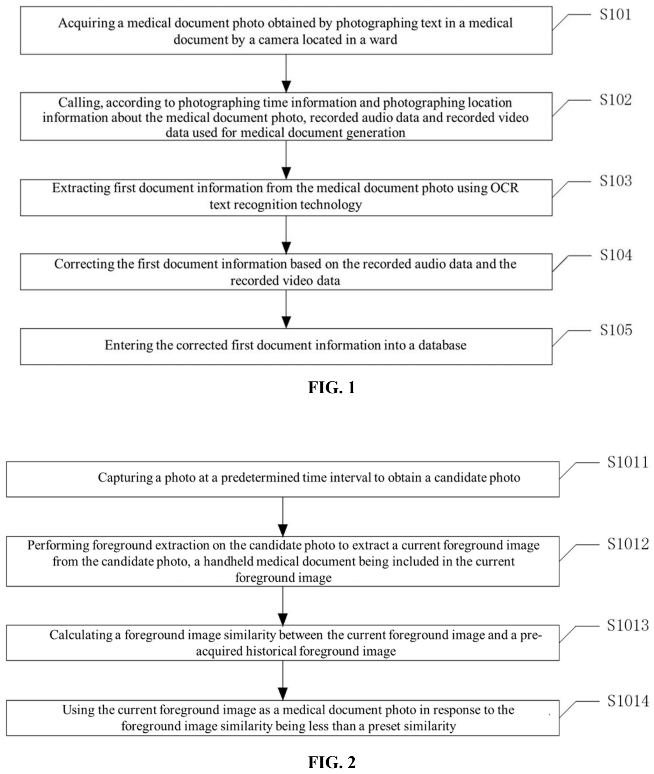

In view of the above situation, the present invention provides a method for entering medical information, as shown in FIG. 1, comprising the steps of:
S101: acquiring a medical document photo obtained by photographing text in a medical document by a camera located in a ward;
S102: calling, according to photographing time information and photographing location information about the medical document photo, recorded audio data and recorded video data used for medical document generation;
S103: extracting first document information from the medical document photo using OCR text recognition technology;
S104: correcting the first document information based on the recorded audio data and the recorded video data; and
S105: entering the corrected first document information into a database.

The solution according to the present invention is applicable to scenarios where doctors make the rounds of the wards, in which doctors need to write text on medical documents anytime and anywhere, thus the text is written with low normativity.

In step S101, the camera can be carried by a doctor or installed at a fixed position in the ward. If a portable camera carried by a doctor is used for photographing, the photographic quality and the remaining battery should be taken into consideration. Therefore, when choosing a portable camera, it is necessary to preferentially consider using a camera that can capture scenes enough for subsequent image analysis and to consider setting up a remaining battery alarm function. Correspondingly, a fixed camera installed at a fixed position in the ward is provided with an electric power transmission line, thus it is unnecessary to consider the problem of power endurance, and it is only necessary to consider the problem of definition.

Generally speaking, a portable camera may be carried in coat pockets of the doctors, because doctors all place medical documents in front of them for record-keeping when keeping records in the medical documents. In this way, the portable camera can conveniently complete acquisition of images. Correspondingly, if the camera is a fixed camera installed at a fixed position in the ward, the photographing angle should be taken into consideration (the doctor or other objects may block the camera from capturing photos of medical documents). Therefore, in general, if a fixed camera is employed, it should be considered that multiple cameras should be set up in a room (usually one camera can be set up in each of the four corners of the ceiling of a room). Meanwhile, the resolution of the cameras should also be taken into consideration. This is mainly because the cameras in the corners of the ceiling, which are far away from medical documents, will affect the subsequent OCR recognition if they do not have enough photographic definition.

During photographing, the system can perform real-time image quality analysis using computer vision technology, and provide real-time feedback of image quality information to a user in combination with a deep-learning image quality evaluation model to guide the user to capture high-quality images. The captured pictures can be first subjected to pre-processing, including operations such as denoising, binarization, dilation, and erosion, so as to improve recognizability of text in the images. After OCR recognition is completed, the recognition results can further be subjected to post-processing, mainly including semantic modification using a deep learning-based language model, to improve the accuracy of the recognition results.

Generally speaking, capturing of medical document photos can be performed regularly or executed after being triggered. Specifically, the doctor can manually trigger a controller of the camera to control the camera to photograph (the controller can be provided on the camera, or a remote controller can be used for photographing), or photographing can be performed at an interval of a time period (usually a few seconds).

Specifically, as shown in FIG. 2, the step S101 can be implemented as follows:
S1011: capturing a photo at a predetermined time interval to obtain a candidate photo;
S1012: performing foreground extraction on the candidate photo to extract a current foreground image from the candidate photo, a handheld medical document being included in the current foreground image;
S1013: calculating a foreground image similarity between the current foreground image and a pre-acquired historical foreground image; and
S1014: using the current foreground image as a medical document photo if the foreground image similarity is less than a preset similarity.

In step S1011, the camera performs photographing at a certain time interval so as to obtain a candidate photo. In this step, the camera is a portable camera set on the body of the doctor. A photo of a medical document held in the hand of the doctor should be captured in the candidate photo.

Then, in step S1012, it is necessary to perform foreground extraction on the candidate photo. Since it is very certain that the object of foreground extraction is a medical document, the following two modes can be selected in order to improve the efficiency and accuracy of foreground extraction. In the first mode, a recognition mark is added to a medical document, and the recognition mark can be a pattern, image, or other mark that is not common in a hospital scenario. For example, the mark can be a black star, and the star is located in the upper right corner of the medical document. Then it can be determined, by means of image recognition, whether the mark is present in the candidate photo. If it is present, a predetermined area on the lower left side of the mark is used as a foreground image. In the specific implementation, the mark can be designed as a direction-pointing mark. In this way, it can be ensured that the mark is facing a specific direction (e.g., facing up), and a region on its lower left side or at a position relative thereto is the foreground image.

In other words, the step S1012 can be implemented as follows:
detecting whether a recognition mark is present in the current candidate photo; and
if it is present, extracting a sampled image from the current candidate photo using positional information and shape information of the recognition mark.

Specifically, the positional information and shape information can reflect the size and pointing direction of the recognition mark. The size of the foreground image can be reflected according to the size of the recognition mark (or the dimension ratio of the foreground image to the recognition mark is fixed, thus the size of the recognition mark can reflect the size of the foreground image), which can thus assist in the positioning of the foreground image. The pointing direction reflects the relative positions of the recognition mark and the foreground image.

In the second mode, foreground extraction can be performed by using a deep learning algorithm, and the deep learning algorithm is mainly composed of structures such as a convolutional neural network (CNN)-based segmentation model, an automatic feature extraction mechanism, and a network model combined with an encoder-decoder structure. The foreground extraction is performed in this mode, mainly because a medical document has a relatively normative format architecture. Specifically, the content in the medical document is not simply blank paper, but is composed of a plurality of block diagrams and text in standard formats that are designed in advance according to inquiries rules of the doctors. Specifically, different positions in the medical document are respectively provided with prompt text and block diagrams/tables, and the prompt text and block diagrams/tables are used to normalize the medical inquiry records of the doctor, Therefore, in the case of a certain degree of normalization, foreground extraction can be performed by using the above deep learning method (when performing foreground extraction, these normalized contents can be used for positioning). Meanwhile, since other content similar to the normalized content can hardly be encountered in a hospital scenario, misrecognition by the deep learning algorithm will not be caused.

In the specific implementation, the above two modes can be used in combination. In other words, the first mode is used first for positioning and sampling box extraction, and then the second mode is used for specific extraction, thereby avoiding the problem of consuming excessive computing power due to excessive sampling boxes in the second mode.

In the specific implementation, the step S1012 can be implemented as follows:
step 10121: detecting whether a recognition mark is present in the current candidate photo;
step 10122: if it is present, determining a sampled image of the current candidate photo using positional information and shape information of the recognition mark; and
step 10123: inputting the sampled image into a pre-trained foreground extraction model to acquire a current foreground image.

Since the sampled image is positioned based on the recognition mark, the main function of the sampled image is to narrow a search range within which processing is performed by the foreground extraction model. Since many images are produced by photographing, it is preferable to use step 10121 to step 10123 for initial screening of foreground images. In some case, a sampling box of the foreground extraction model in step 10123 can be similar to the size of the sampled image extracted in step 10122. In this way, the sampling range can be further reduced. Specifically, the sampling box size and step size can be determined according to the current remaining computing power. The remaining computing power correlates negatively with the sampling box size, and the remaining computing power correlates negatively with the step size.

In step S1013, the main task is to calculate the similarity between the current foreground image and a historical foreground image. Generally speaking, there are two types of historical foreground images. One type is a completely blank medical document (not handwritten by the doctor), and the medical document only has normalized prompt text and block diagrams/tables. The other type is a medical document photographed a time period ago, in general, a foreground image extracted from a medical document photo captured 5 to 10 seconds ago.

When the historical foreground image is a completely blank medical document, it can be known, by calculating the similarity, whether text is handwritten by the doctor in the medical document (if handwritten text appears in the medical document, the similarity decreases, and the similarity is lower when there are more characters). However, this mode has certain deficiencies, mainly in that the similarity becomes lower and lower as content handwritten by the doctor increases. In other words, in the handwriting process of the doctor, continuous triggering of step S1014 can occur, resulting in a decrease in precision. In order to avoid this problem, the similarity can be calculated by using multiple continuous photos. In other words, one candidate photo is acquired at an interval of a time period (N seconds, e.g., 3 seconds), and then the similarity is calculated using the candidate photo. Since the doctor continuedly writes content in the medical document, the similarity of candidate photos acquired subsequently will decrease continuously. When the similarity of the candidate photos acquired within a time period (M seconds, e.g., 10 seconds, or a period which is 3 to 5 times N seconds) no longer decreases, or no foreground image can be extracted, the current foreground image with the lowest similarity is used as the medical document photo in step S1014.

When the historical foreground image is a medical document photographed a time period ago, the similarity to be calculated mainly refers to the similarity between foreground images of two consecutively captured candidate photos, which reflects whether the doctor performed a handwriting action during this time period. If a lot of content was handwritten by the doctor during this time period, the execution of step S1014 will be triggered. In order to avoid occurrence of misidentification, if no foreground image can be recognized from the current candidate photo, a foreground image from a previously acquired candidate photo should be used as the medical document photo.

Although there may be persons and other things in the background part of the medical document photo, and there may be normalized tables and text in the foreground image, these parts which are not text written by the doctor can be extracted for subsequent OCR recognition during extraction of the foreground image.

Specifically, the step S102 can be implemented as follows:
S1021: determining, according to the photographing location information about the medical document photo, a candidate room where the medical document photo is captured;
S1022: calculating a score of a reference picture of each candidate room according to a room mark recorded in a background image of the medical document photo by means of visual positioning;
S1023: determining a candidate room with a score exceeding a preset value as a target room; and
S1024: selecting, from video data recorded in the target room, a target recorded audio segment as recorded audio data to be called for the medical document generation, and a target video segment as recorded video data to be called for the medical document generation, according to the photographing time information about the medical document photo.

In step S1021, the candidate room where the medical document photo is captured can be determined by means of GPS positioning or BeiDou positioning, but the floor where the doctor is located cannot be determined by using these two positioning modes. Thus, rooms with the same latitude and longitude coordinates (e.g., the leftmost room on each floor) can all be identified as candidate rooms. Of course, in addition to the above two positioning modes, other positioning technologies can also be used in implementation. Specifically, for example, Wi-Fi positioning or the like can be used for positioning, but it should be noted that Wi-Fi positioning also involves deviations, and even if Wi-Fi positioning is combined with GPS/BeiDou positioning, there may be errors. Although other positioning technologies such as strapdown inertial guidance and infrared technologies exist in the current technical environment, these technologies do not have high practical value in medical scenarios, are only implementable theoretically, involve high actual cost, and can hardly be used in practice. Therefore, in this solution, only GPS or BeiDou positioning technology is usually used. Then, all rooms corresponding to the coordinates can be determined as candidate rooms.

The step S1022 can be performed by means of visual positioning, because a medical document photo can inevitably be acquired in this solution. When a medical document photo is acquired, it is generally impossible that the whole image is a medical document, and there are also some background images. Thus, different room marks are set on different floors to assist in positioning. Then, when the room mark is photographed, the floor of the medical document photo can be determined (the room mark will be photographed in the background image). Of course, a more complex method can also be used for processing. In other words, a visual positioning technology used on automatic robots is utilized to perform direct image coordinate conversion and indirect image coordinate conversion by using the captured photo and to map the converted coordinates to a predetermined virtual three-dimensional space (where the three-dimensional space is established according to the characteristics of the hospital building), whereby accurate positioning can be carried out. However, this positioning mode is not practical, mainly because it consumes excessive computing power. For portable devices, excessive occupation of computing power will make it impossible to analyze the captured photos in time, but it is certainly undeniable that this mode can achieve technical purposes.

After the room mark is recognized by means of visual positioning, a reference score of a reference picture of each candidate room can be determined (the reference score mainly reflects a probability of presence of the user in each candidate room when the medical document photo is captured). Here, the reference picture of the candidate room (picture including a room mark) is acquired in advance. One reference picture can be saved for each room (each room has a different room mark), or rooms on each floor can share one reference picture (rooms on the same floor have the same room mark, and rooms on different floors have different room marks). After the score is determined, a candidate room with the highest score may be used as a target room in step S1023.

By arranging a camera in each room in advance (usually at a corner of the room ceiling), it is possible in step S1024 to use an audio data segment captured at a time consistent with the photographing time information about the medical document photo as recorded audio data to be called for the medical document generation, and use a video segment captured at a time consistent with the photographing time information about the medical document photo as recorded video data used for the medical document generation, from among content acquired by the camera. The recorded audio data and recorded video data used for medical document generation here reflect language and body movements during record-keeping in the medical document by the doctor (which may not be the language and body movements of the doctor who wrote in the medical document), respectively.

Specifically, step S104 can be implemented as follows:
S1041: performing speech recognition on the recorded audio data to obtain speech-recognized text;
S1042: analyzing body movements of a person in the recorded video data to obtain body movement characteristic data;
S1043: extracting referential second document information corresponding to the speech-recognized text from a pre-established historical speech-recognized text - referential second document information comparison table based on the speech-recognized text;
S1044: extracting referential third document information corresponding to the body movement characteristic data from a pre-established historical body movement characteristic data - referential third document information comparison table based on the body movement characteristic data; and
S1045: correcting the first document information based on the referential second document information and the referential third document information.

In step S1041, a conventional speech-to-text recognition can be mainly carried out (recognition of mandarin is sufficient). In some cases, if a region where this method is implemented can already be determined, it is possible to consider adding a dialect speech recognition technology. Specifically, this text conversion process relies mainly on acoustic models and language models. Here, acoustic models are responsible for converting speech signals into phoneme or word-level representations, while language models convert these representations into final text outputs. In the training stage, the system needs to establish acoustic models and language models by analyzing a large amount of speech data. In the recognition stage, the system can automatically recognize real-time speech and generate textual results.

In step S1042, recognition of body movements is mainly performed, which can specifically be implemented using human keypoint detection, deep learning models, and the like. Considering that a medical scenario is indoors and has sufficient light with little interference, general techniques can be used. Since the body movement recognition results (body movement characteristic data) are directly used in the subsequent step for search in the table, it is not necessary to give a clear meaning to each body movement recognition result, as long as they can be correlated. For clarity purposes, only two examples are given here for illustration. A body movement recognition result can mean nodding with hands behind the back, which can indicate that the patient is in a stage that meets the expectation of treatment. For another example, a body movement recognition result can mean a specific body stretching movement (e.g., horizontal arm extension in a forward bending state, thigh raising, or the like), which indicates that the doctor is guiding the patient to perform a certain movement for verifying rehabilitation.

Steps S1043 and S1044 have similar contents, both of which involve lookup in an established table. For example, contents in the historical speech-recognized text - referential second document information comparison table and the historical body movement characteristic data - referential third document information comparison table are based on information that has been historically tested and deemed correct by doctors, and thus have good reference value.

It is deemed that in the historical speech-recognized text - referential second document information comparison table, the historical speech-recognized text and the referential second document information corresponding to each other are a text recognition result obtained by speech recognition in step S1041 and text obtained after the text recognition result has been confirmed/modified, respectively. Correspondingly, in the historical body movement characteristic data - referential third document information comparison table, the historical body movement characteristic data and the referential third document information corresponding to each other are body movement characteristic data historically recognized by step S1042 and text content obtained after doctors have recognized the body movement characteristic data or viewed the recorded video data, respectively.

It should be noted that the referential second document information and the referential third document information usually do not directly replace the first document information, but the referential second document information and the referential third document information can reflect which kind/piece of first document information is the closest to the reality. Specifically, the first document information extracted from the medical document photo by using OCR text recognition technology is not unique (the model outputs each text arrangement mode and a corresponding probability). Therefore, in step S103, the model can be allowed to selectively output 3 to 5 results with the highest probability (candidate first document information), and then one final result that is most logically (usually logically in linguistics) similar to the referential second document information and the referential third document information is selected from these 3 to 5 results by using correlations between the referential second and third document information and these 3 to 5 results.

For example, if the body movement recognition result means a specific body stretching movement, the probability of using a result, included in the multiple results, in which rehabilitation recommendations are described as the final result can be increased. In some cases, the content recorded by doctors in documents may not include rehabilitative movements due to different attributes of the documents (for example, the content recorded in the documents is viewed by the doctors themselves, and there is no need to record rehabilitative movements). Thus, semantic recognition should be used to calculate semantic correlations of different candidate first document information with the second document information and the third document information, respectively, and then it is determined, according to the semantic correlations, which candidate first document information is the most accurate. If the content related to medication administration requirements in the rehabilitation stage is recorded in a certain piece of candidate first document information, although only body movements appear in the third document information in which there is no action of taking medication, it should also be deemed that there is a relatively strong correlation between this candidate first document information and the third document information, because the objects described in both of them are related to rehabilitation. The probability of using this candidate first document information as the final result should be increased.

Similarly, the processing mode for the second document information is the same as the processing mode for the third document information. Text contents are not required to be completely correlated, as long as they are strongly correlated to each other, for example, the objects described are correlated to each other. Since fewer types of communication content appear in hospitals, this mode can be adopted only in hospital environments.

Specifically, a certain piece of candidate first document information is selected as the final result by using the second document information and the third document information. In addition, the final output result can also be modified by using the second document information and the third document information, but this part of modification is not a substantial variation and is merely a formal fine-adjustment, and thus will not be explained deeply.

In other words, step S1045 can be implemented as follows:
step 10451: calculating a first semantic correlation between each first document information (candidate first document information) and the referential second document information;
step 10452: calculating a second semantic correlation between each first document information (candidate first document information) and the referential third document information;
step 10453: selecting target first document information from multiple pieces of first document information according to the first semantic correlation and the second semantic correlation; and
step 10454: correcting the target first document information by using the referential second document information and the referential third document information.

In step S105, the first document information entered into the database is the target first document information.

Of course, when performing OCR recognition, it is possible to consider recognizing text in a preprocessed image using an open-source OCR engine such as Tesseract, or a deep learning-based OCR model such as CRNN or Attention OCR.

Specifically, a semantic correlation between each result and the referential second document information and a semantic correlation between each result and the referential third document information can be calculated respectively, then a semantic similarity parameter of each result can be calculated by means of weighted averaging, and then a result with the maximal semantic similarity parameter can be used as a correction result of step S104 (the first document information in step S104). Finally, the correction result can be entered into the database.

However, in practical processing, there can be little difference in the magnitude of the semantic similarity parameter between the candidate first document information with the highest semantic similarity parameter and the candidate first document information with the second and third highest semantic similarity parameters. In this case, it is possible to consider performing processing in the follow manner. In other words, step S105 can be implemented as follows:
S1051: if there are multiple pieces of corrected first document information, retrieving medical information of a patient corresponding to the medical document photo;
S1052: displaying the medical information and the multiple pieces of corrected first document information in the terminal of the doctor to select one of the multiple pieces of corrected target first document information as final first document information according to a selection instruction received by the terminal of the doctor; and
S1053: storing the final first document information in the database and updating the historical speech-recognized text - referential second document information comparison table and the historical body movement characteristic data - referential third document information comparison table based on the corrected final first document information.

In other words, when it is not possible to distinguish between several pieces of candidate first document information with higher semantic similarity parameters by using the referential second document information and the referential third document information, the historical medical information of the patient can be used for reference. The medical information can for example include the personal information (height, age, weight, etc.) of the patient, historical medical treatment information (historical illness, treatment status, etc.), current diagnosis and treatment information, and the like.

After the medical information is acquired, this information and the corrected first document information can be displayed on the terminal of the doctor, thereby allowing the doctor to manually select/manually modify a certain piece of first document information as the final first document information. Thereafter, the first document information selected/modified by the doctor can be stored in the database. Meanwhile, the tables in the database can also be updated so that the next recognition will be performed more accurately. Of course, from a practical point of view, the terminal of the doctor should have certain permissions to view the tables in the database so as to correct wrong information in a timely manner.

The text that is finally stored in the database is preferably converted into a structured data format, such as JSON, for facilitating subsequent processing and use.

The method according to the present invention as a whole has the following advantages:
1. High efficiency: the technology according to the present invention enables users to fill out forms by uploading pictures (e.g., examination forms, test forms, etc.) by using OCR technology and big data model technology, which greatly saves the time required for filling out the forms by the users. Compared with traditional manual filling modes, our method can reduce the time required for filling out forms by more than 70%, thereby greatly improving the efficiency of data entry.
2. Improved accuracy: since the present invention employs speech recognition results and body recognition results to assist in recognition, and also employs a series of technical means such as image quality enhancement, image preprocessing and post-processing, OCR recognition, and data structuring, the accuracy of the recognition results can be effectively improved. According to the experimental data, the present invention significantly improves the accuracy of data entry as compared with traditional OCR technologies.
3. User-friendliness: the technology according to the present invention also provides data storage and presentation functions to present the OCR results to users in the form of charts or sheets, so that the users can intuitively see the OCR results, which greatly improves the user experience. Meanwhile, the present invention also enables direct storage of the OCR results in the database to facilitate subsequent data query and use by the users.
4. Environmental-friendliness and reduced labor intensity: traditional data entry modes require a lot of paper and manual work, which not only affects the environment but also has high labor intensity. The method of the present invention is completely electronic, which is not only environmentally friendly, but also greatly reduces labor intensity.

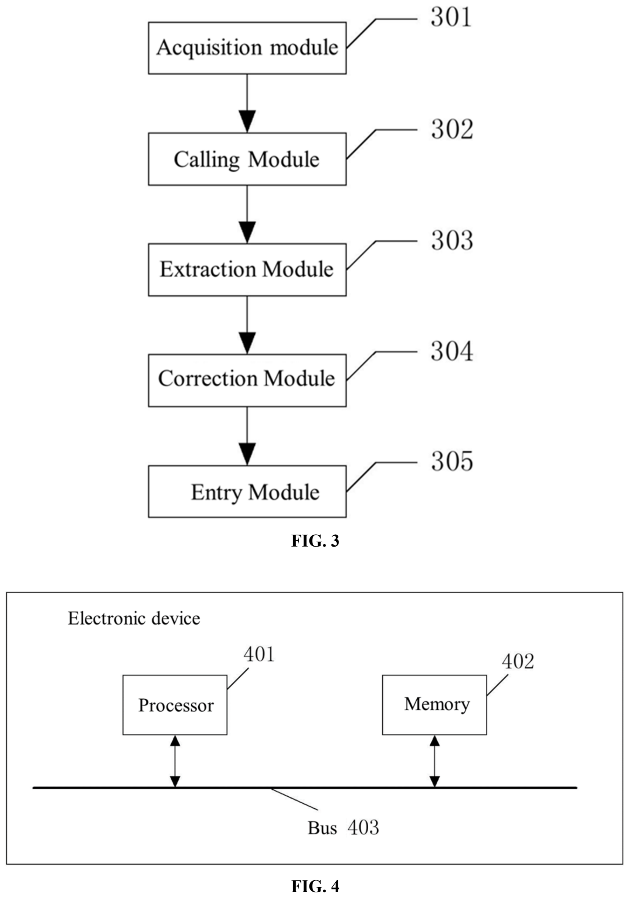

Based on the same technical concept, the present invention further provides an apparatus for entering medical information. As shown in FIG. 3, the apparatus comprises:
an acquisition module 301 configured to acquire a medical document photo obtained by photographing text in a medical document by a camera located in a ward;
a calling module 302 configured to call, according to photographing time information and photographing location information about the medical document photo, recorded audio data and recorded video data used for medical document generation;
an extraction module 303 configured to extract first document information from the medical document photo using OCR text recognition technology;
a correction module 304 configured to correct the first document information based on the recorded audio data and the recorded video data; and
an entry module 305 configured to enter the corrected first document information into a database.

Optionally, the camera is a portable camera set on a body of a doctor; and the acquisition module 301, which is configured to acquire the medical document photo obtained by photographing text in the medical document by the camera located in the ward, is specifically configured to:
capture a photo at a predetermined time interval to obtain a candidate photo;
perform foreground extraction on the candidate photo to extract a current foreground image from the candidate photo, a handheld medical document being included in the current foreground image;
calculate a foreground image similarity between the current foreground image and a pre-acquired historical foreground image; and
use the current foreground image as a medical document photo if the foreground image similarity is less than a preset similarity.

Optionally, the calling module 302, which is configured to call, according to photographing time information and photographing location information about the medical document photo, recorded audio data and recorded video data used for medical document generation, is specifically configured to:
determine, according to the photographing location information about the medical document photo, a candidate room where the medical document photo is captured;
calculate a score of a reference picture of each candidate room according to the medical document photo by means of visual positioning;
determine a candidate room with a score exceeding a preset value as a target room; and
select, from video data recorded in the target room, a target recorded audio segment as recorded audio data to be called for the medical document generation, and a target video segment as recorded video data to be called for the medical document generation, according to the photographing time information about the medical document photo.

Optionally, the correction module 304, which is configured to correct the first document information based on the recorded audio data and the recorded video data, is specifically configured to:
perform speech recognition on the recorded audio data to obtain speech-recognized text;
analyze body movements of a person in the recorded video data to obtain body movement characteristic data;
extract referential second document information corresponding to the speech-recognized text from a pre-established historical speech-recognized text - referential second document information comparison table based on the speech-recognized text;
extract referential third document information corresponding to the body movement characteristic data from a pre-established historical body movement characteristic data - referential third document information comparison table based on the body movement characteristic data; and
correct the first document information based on the referential second document information and the referential third document information.

Optionally, the entry module 305, which is configured to enter the corrected first document information into a database, is specifically configured to:
retrieve, if there are multiple pieces of corrected first document information, medical information of a patient corresponding to the medical document photo;
display the medical information and the multiple pieces of corrected first document information in the terminal of the doctor to select one of the multiple pieces of corrected first document information as final first document information according to a selection instruction received by the terminal of the doctor; and
store the final first document information in the database and update the historical speech-recognized text - referential second document information comparison table and the historical body movement characteristic data - referential third document information comparison table based on the corrected final first document information.

FIG. 4 is a schematic structural diagram of an electronic device according to an embodiment of the present invention, comprising: a processor 401, a memory 402, and a bus 403. The memory 402 stores machine-readable instructions executable by the processor 401. When the electronic device runs the above-mentioned method for entering medical information, the processor 401 communicates with the memory 402 via the bus 403, and the processor 401 executes the machine-readable instructions to execute the method steps described in the embodiment.

An embodiment of the present invention further provides a computer-readable storage medium. A computer program is stored on the computer-readable storage medium. The computer program is run by a processor to execute the method steps described in the embodiment.

It will be clearly appreciated by those skilled in the art that, for convenience and brevity of the description, specific operating processes of the apparatus, the electronic device, and the computer-readable storage medium described above may be performed with reference to the corresponding processes in the foregoing embodiment of the method and will not be described in detail herein.

In several embodiments according to the present invention, it should be understood that the disclosed method, apparatus, electronic device, and computer-readable storage medium can be implemented in other ways. The embodiments of the apparatus described above are merely illustrative in nature. For example, the modules are divided only by logical functions, and additional division modes can be adopted in practical implementation. For another example, multiple modules or components can be combined or integrated into another system, or some features may be omitted or not executed. In addition, the mutual coupling or direct coupling or communication connection illustrated or discussed can be implemented via indirect coupling or communication connection between some communication interfaces, apparatuses, or units, which can be electronic, mechanical, or in other forms.

The units described as separate components can be or not be separated physically. The components illustrated as units can be or not be physical units. In other words, they can be located at one place or they can be distributed onto multiple network units. Some or all of the units can be selected as actually required to fulfill the purposes of the solutions of the present embodiments.

Besides, the individual functional units in the embodiments of the present invention can be integrated into one processing unit, or each of the units can be physically stand-alone, or two or more of the units can be integrated into one unit.

When implemented in the form of a software functional unit and sold or used as an independent product, the functions can be stored in a nonvolatile computer-readable storage medium executable by a process. Based on such understanding, a technical solution of the present invention essentially, or its part contributing to the prior art, or a part of the technical solution can be embodied in the form of a software product. The computer software product is stored in a storage medium, and includes a number of instructions for causing a computer device (which can be a personal computer, a server, a network device, or the like) to execute all or some of the steps of the methods described in the various embodiments of the present invention. The preceding storage medium includes any medium that can store program code, such as a USB flash disk, a mobile hard disk, a read-only memory (ROM), a random access memory (RAM), a magnetic disk, or an optical disk.

Finally, it should be noted that the embodiments described above are merely specific embodiments of the present invention, which are intended to illustrate the technical solutions of the present invention and not intended to limit the present invention, and to which the scope of protection of the present invention is not limited. Although the present invention has been illustrated in detail with reference to the foregoing embodiments, it should be understood by those skilled in the art that the technical solutions described in the foregoing embodiments can be modified, or variations thereof can be readily envisaged, or some of the technical features thereof can be equivalently replaced by those skilled in the art within the technical scope disclosed in the present invention. Such modifications, variations, or replacements do not cause the essence of the corresponding technical solutions to depart from the spirit and scope of the technical solutions of the embodiments of the present invention, and are therefore intended to be encompassed within the scope of protection of the present invention. Therefore, the scope of protection of the present invention should be defined by the scope of the appended claims.

## Claims

1. A method for entering medical information, **characterized by** comprising:
acquiring a medical document photo obtained by photographing text in a medical document by a camera located in a ward;
calling, according to photographing time information and photographing location information about the medical document photo, recorded audio data and recorded video data used for a medical document generation;
extracting first document information from the medical document photo using OCR text recognition technology;
correcting the first document information based on the recorded audio data and the recorded video data; and
entering the corrected first document information into a database.

2. The method according to claim 1, wherein the camera is a portable camera arranged on a body of a doctor; and the acquiring the medical document photo obtained by photographing text in the medical document by the camera located in the ward comprises:
capturing a photo at a predetermined time interval to obtain a candidate photo;
performing foreground extraction on the candidate photo to extract a current foreground image from the candidate photo, a handheld medical document being included in the current foreground image;
calculating a foreground image similarity between the current foreground image and a pre-acquired historical foreground image; and
using the current foreground image as a medical document photo in response to the foreground image similarity being less than a preset similarity.

3. The method according to claim 1, wherein the calling, according to the photographing time information and the photographing location information about the medical document photo, the recorded audio data and the recorded video data used for the medical document generation comprises:
determining, according to the photographing location information about the medical document photo, a candidate room where the medical document photo is captured;
calculating a score of a reference picture of each candidate room according to the medical document photo by means of visual positioning;
determining a candidate room with a score exceeding a preset value as a target room; and
selecting, from video data recorded in the target room, a target recorded audio segment as the recorded audio data to be called for the medical document generation, and a target video segment as recorded video data to be called for the medical document generation, according to the photographing time information about the medical document photo.

4. The method according to claim 3, wherein the correcting the first document information based on the recorded audio data and the recorded video data comprises:
performing speech recognition on the recorded audio data to obtain speech-recognized text;
analyzing body movements of a person in the recorded video data to obtain body movement characteristic data;
extracting referential second document information corresponding to the speech-recognized text from a pre-established historical speech-recognized text - referential second document information comparison table based on the speech-recognized text;
extracting referential third document information corresponding to the body movement characteristic data from a pre-established historical body movement characteristic data - referential third document information comparison table based on the body movement characteristic data; and
correcting the first document information based on the referential second document information and the referential third document information.

5. The method according to claim 4, wherein the entering the corrected first document information into the database comprises:
retrieving medical information of a patient corresponding to the medical document photo in response to multiple pieces of corrected first document information being present;
displaying the medical information and the multiple pieces of corrected first document information in a terminal of the doctor to select one of the multiple pieces of corrected first document information as final first document information according to a selection instruction received by the terminal of the doctor; and
storing the final first document information in the database and updating the historical speech-recognized text - referential second document information comparison table and the historical body movement characteristic data - referential third document information comparison table based on the corrected final first document information.

6. The method according to claim 2, wherein the performing foreground extraction on the candidate photo to extract the current foreground image from the candidate photo comprises:
detecting whether a recognition mark is present in the current candidate photo;
extracting a sampled image from the current candidate photo using positional information and shape information of the recognition mark in response to the recognition mark being present in the current candidate photo; and
inputting the sampled image into a pre-trained foreground extraction model to acquire the current foreground image.

7. The method according to claim 4, wherein the correcting the first document information based on the referential second document information and the referential third document information comprises:
calculating a first semantic correlation between each first document information and the referential second document information;
calculating a second semantic correlation between each first document information and the referential third document information;
selecting target first document information from multiple pieces of first document information according to the first semantic correlation and the second semantic correlation; and
correcting the target first document information by using the referential second document information and the referential third document information.

8. An apparatus for entering medical information, **characterized by** comprising:
an acquisition module (301) configured to acquire a medical document photo obtained by photographing text in a medical document by a camera located in a ward;
a calling module (302) configured to call, according to photographing time information and photographing location information about the medical document photo, recorded audio data and recorded video data used for medical document generation;
an extraction module (303) configured to extract first document information from the medical document photo using OCR text recognition technology;
a correction module (304) configured to correct the first document information based on the recorded audio data and the recorded video data; and
an entry module (305) configured to enter the corrected first document information into a database.

9. An electronic device, **characterized by** comprising: a processor (401), a memory (402), and a bus (403), wherein the memory (402) stores machine-readable instructions executable by the processor (401), and when the electronic device is running, the processor (401) communicates with the memory (402) via the bus (403), and the machine-readable instructions are executed by the processor (401) to execute the steps of the method according to any one of claims 1 to 7.

10. A computer-readable storage medium, **characterized in that** a computer program is stored on the computer-readable storage medium, and the computer program is run by a processor to execute the steps of the method according to any one of claims 1 to 7.
